# EUROPEAN PATENT SPECIFICATION

(11) **EP 3 782 679 B1**
(45) Date of publication and mention of the grant of the patent: **02.07.2025**
(21) Application number: 19787708.7
(22) Date of filing: 18.04.2019
(51) Int. Cl.: A61M 5/46, A61M 5/28, A61M 5/32

(54) **INJECTION NEEDLE AND INJECTION DEVICE**
INJEKTIONSNADEL UND INJEKTIONSVORRICHTUNG
AIGUILLE D'INJECTION ET DISPOSITIF D'INJECTION

(30) Priority: 20.04.2018 JP 2018081378
(43) Date of publication of application: 24.02.2021
(73) Proprietor: Asti Corporation, Hamamatsu-shi, Shizuoka 432-8056 (JP)
(72) Inventor: INOH Akinori, Hamamatsu-shi, Shizuoka 432-8056 (JP); NONAKA Isamu, Hamamatsu-shi, Shizuoka 432-8056 (JP); OGAI Noriyuki, Hamamatsu-shi, Shizuoka 432-8056 (JP)
(74) Representative: Grünecker Patent- und Rechtsanwälte PartG mbB
(86) International application number: PCT/JP2019/016607
(87) International publication number: WO 2019/203298

(56) References cited:
- EP-A1- 2 633 882
- EP-A1- 2 633 882
- JP-A- 2014 042 669
- JP-U- 3 194 715
- KR-A- 20110 007 512
- KR-A- 20120 020 216
- KR-A- 20120 020 216
- US-A1- 2018 093 049

## Description

### TECHNICAL FIELD

The present invention relates to an injection needle and an injection device for performing prevention and treatment of various diseases, for example by subcutaneous or intradermal administration of an objective substance, and more particularly, the present invention relates to the injection needle and the injection device, of which puncture depth of a needle can be adjusted.

### BACKGROUND ART

As an example disclosing an injection needle and an injection device, there is a Patent Document 1. A percutaneous administration device as described in Patent Document 1 is composed of a medicinal solution injection needle and a syringe. The medicinal solution injection needle comprises a medicinal solution injection needle body made of resin. The medicinal solution injection needle body has a cylindrical shape, and a hollow portion has been formed on the side of the base end thereof. Moreover, the medicinal solution injection needle body has three penetration holes formed therein, into which needles are inserted and fixed, respectively.

Meanwhile, the syringe is composed of a syringe body and a plunger. The top end side of the syringe body is inserted into the hollow portion of the medicinal solution injection needle body, whereby the medicinal solution injection needle is attached to the syringe.

Thus, when the plunger is depressed so as to discharge a medicinal solution from the inside of the syringe body, the medicinal solution enters from the hollow portion of the medicinal solution injection needle body into the inside of the needle, so as to be injected subcutaneously or intradermally from the top end of the needle.

Moreover, before using, the medicinal solution injection needle has been covered by a needle holder. Further relevant prior art is described in EP 2 633 882 A1, KR 2012 0020216 A and KR 2011 0007512 A.

### REFERENCE DOCUMENTS OF CONVENTIONAL ART

### PATENT DOCUMENT(S)

Patent Document 1: Official Gazette, Japan Patent No. 5890430.

### SUMMARY OF THE INVENTION

### PROBLEMS TO BE SOLVED BY INVENTION

However, the above structure of the conventional art has the following problem:
The subcutaneous or intradermal puncture depth of the needle will vary and is not constant. On the other hand, with regard to the percutaneous administration device as described in Patent Document 1, the protrusion length of the needle fixed in the medicinal solution injection needle body, namely the protrusion length of the needle protruding from the medicinal solution injection needle body, is invariable, and of course, the puncture depth is also invariable. Consequently, in order to cope with various puncture depths, it has been necessary to prepare several types of medicinal solution injection needles, each of which has a different protrusion length of the needle protruding from the medicinal solution injection needle body.

In the light of the above problem, it is an object of the present invention to provide an injection needle and an injection device, of which puncture depth of a needle can be adjusted.

### MEANS TO SOLVE THE PROBLEM

The present invention provides an injection needle having the features defined in claim 1. Further preferred embodiments are defined in the dependent claims.

### EFFECT OF THE INVENTION

As described above, according to Claim 1 , there is the injection needle provided with: a needle; a needle base holding the needle in a state of protruding toward the top end side; and an adjuster disposed to be movable along the axis direction of the needle base on the outer periphery of the needle base, wherein a protrusion length of the needle is adjusted by adjusting a position of the adjuster in the axis direction. Therefore, the protrusion length of the needle can be adjusted, and it is no longer necessary to prepare several types of injection needles, each of which has a different protrusion length as was the case of the conventional art.
Moreover, the adjuster is movable in the axis direction of the needle base by a screw engagement structure. Therefore, the puncture depth of the needle can be adjusted by easy operation. Moreover, the moving amount of the adjuster toward the top end side is limited to a predetermined amount by a stopper mechanism. Therefore, it is possible to prevent the drop-off toward the top end side of the adjuster.

Moreover, the stopper mechanism is composed of a collar portion on the side of the adjuster becoming in contact with an end of a male screw portion on the side of the needle base. Therefore, the stopper mechanism in a simple structure can be composed.

Moreover, according to the injection needle of Claim 2 , with regard to the injection needle as claimed in Claim 1, the top end of the needle is capable of being retracted in the adjuster. Therefore, for example, attachment of a cap after injection is not required, and for example, it is possible to prevent accidental needle pricking caused by double-puncture (wrong puncture after completion of puncture) by users such as physicians or nurses.

Moreover, according to the injection needle of Claim 3 , with regard to the injection needle as claimed in Claim 1, a needle base is provided with a needle-base side reference position marker, and the adjuster is provided with an adjuster side reference position marker. Therefore, the number of rotations of the adjuster can be known accurately according to the positional relation in the rotative direction between the needle-base side reference position marker and the adjuster side reference position marker, whereby the puncture depth of the needle can be adjusted easily and accurately.

Moreover, according to the injection needle of Claim 4 , with regard to the injection needle as claimed in Claim 1, the adjuster is provided with a longitudinally-split groove, so as to allow expansion of the diameter when attaching to the needle base. Therefore, even where the collar portion, which is a part of the stopper mechanism, is provided, it is possible to attach the adjuster to the needle base, and the assembling thereof can be accomplished easily.

Moreover, according to the injection needle of Claim 5 , with regard to the injection needle as claimed in Claim 1, the cross-section shape of the male screw portion of the needle base is in a non-circular shape, and the cross-section shape of an opening of the collar portion of the adjuster is also in a non-circular shape correspondingly, and by matching each position in the rotative direction, the male screw portion becomes capable of penetrating through the inside of the adjuster. Therefore, also with this structure, even where the collar portion, which is a part of the stopper mechanism, is provided, it is possible to attach the adjuster to the needle base, and the assembling thereof can be accomplished easily.

Moreover, according to the injection needle of Claim 6 , with regard to the injection needle as claimed in any one Claim among Claim 1 to Claim 5, the needle is provided in a plural number. Therefore, it is possible to perform injection more efficiently.

Moreover, according to the injection needle of Claim 7, with regard to the injection needle as claimed in Claim 6, a medicinal solution outlet of the needle is oriented inwardly in the radial direction of the needle base. Therefore, the injected medicinal solution will diffuse widely, and simultaneously, will remain in a shallow portion of the skin.

Moreover, according to the injection needle of Claim 8, with regard to the injection needle as claimed in any one Claim among Claim 1 to Claim 7, the needle base is provided with a holding projection. Therefore, for example, when the injection needle is attached to the top end of the syringe, such an attachment work can be performed by holding the holding projection, and accordingly, any unintended rotation of the adjuster can be prevented.

Moreover, according to the injection needle of Claim 9 , with regard to the injection needle as claimed in any one Claim among Claim 1 to Claim 8, the outer periphery of the adjuster is crowned with a cap so as to cover the needle. Therefore, it is possible to prevent the accidental needle pricking.

Moreover, according to the injection needle of Claim 10, with regard to the injection needle as claimed in Claim 9, the cap is fitted in the adjuster, so that the adjuster is capable of being rotated with the rotation of the cap. Therefore, it is possible to rotate the adjuster in a state of being covered by the cap so as to adjust the protrusion amount of the needle. Consequently, it is also possible to prevent the accidental needle pricking.

Moreover, according to the injection needle of Claim 11, with regard to the injection needle as claimed in Claim 10, the adjuster is provided with an engagement projection, and the cap is provided with an engagement opening to be engaged with the engagement projection. Therefore, with the engagement of the engagement opening with the engagement projection, it is possible to rotate the adjuster in a state of being covered by the cap so as to adjust the protrusion amount of the needle. Consequently, it is also possible to prevent the accidental needle pricking.

Moreover, according to the injection needle of Claim 12, with regard to the injection needle as claimed in Claim 9, the cap encloses the adjuster and is fitted in the needle base in a state that the rotation is prohibited. Therefore, for example, when the injection needle is attached to the top end of the syringe, such an attachment work can be performed by holding the cap, and accordingly, any unintended rotation of the adjuster can be prevented.

### BRIEF DESCRIPTION OF DRAWINGS

[Figure 1] Fig. 1 is a plan view of an injection device according to a first arrangement.
[Figure 2] Fig. 2 is a perspective view of an injection needle as seen from the top end side according to the first arrangement.
[Figure 3] Fig. 3 is a perspective view of the injection needle as seen from the base end side according to the first arrangement.
[Figure 4] Fig. 4 is a sectional view as seen by IV-IV of Fig. 2 according to the first arrangement.
[Figure 5] Fig. 5 is a perspective view of needles and a needle base of the injection needle as seen from the top end side according to the first arrangement.
[Figure 6] Fig. 6 is a perspective view of the needles and the needle base of the injection needle as seen from the base end side according to the first arrangement.
[Figure 7] Fig. 7 is a perspective view of an adjuster of the injection needle as seen from the top end side according to the first arrangement.
[Figure 8] Fig. 8 is a perspective view of the adjuster of the injection needle as seen from the top end side according to the first arrangement.
[Figure 9] Fig. 9 is a longitudinal sectional view of the injection needle in a state that the protrusion length of the needle is decreased by moving the adjuster forward according to the first arrangement.
[Figure 10] Fig. 10 is a longitudinal sectional view of the injection needle in a state that the needles are retracted in the adjuster by further moving the adjuster forward according to the first arrangement.
[Figure 11] Fig. 11 is a perspective view of the injection needle in a state that the needles are covered by attaching a cap to the top end side according to the first arrangement.
[Figure 12] Fig. 12 is a sectional view as seen by XII-XII of Fig. 11 according to the first arrangement.
[Figure 13] Fig. 13 is a longitudinal sectional view of an injection needle in a state that an adjuster moves rearward to the base end according to a second arrangement. The second arrangement is an embodiment of the present invention.
[Figure 14] Fig. 14 is a longitudinal sectional view of the injection needle in a state that the adjuster moves forward to the top end according to the second arrangement.
[Figure 15] Fig. 15 is a perspective view of the injection needle as seen from the top end side according to the second arrangement.
[Figure 16] Fig. 16 is a plan view of an injection device according to a third arrangement.
[Figure 17] Fig. 17 is a perspective view of an injection needle as seen from the top end side, in a state that an adjuster moves rearward to the base end according to the third arrangement.
[Figure 18] Fig. 18 is a perspective view of the injection needle as seen from the base end side, in a state that the adjuster moves rearward to the base end according to the third arrangement.
[Figure 19] Fig. 19 is a sectional view as seen by XIX-XIX of Fig. 17 according to the third arrangement.
[Figure 20] Fig. 20 is a perspective view of the injection needle as seen from the top end side, in a state that the adjuster moves forward to the top end according to the third arrangement.
[Figure 21] Fig. 21 is a perspective view of the injection needle as seen from the base end side, in a state that the adjuster moves forward to the top end according to the third arrangement.
[Figure 22] Fig. 22 is a sectional view as seen by XXII-XXII of Fig. 20 according to the third arrangement.
[Figure 23] Fig. 23 is a perspective view of the adjuster of the injection needle as seen from the top end side according to the third arrangement.
[Figure 24] Fig. 24 is a perspective view of the adjuster of the injection needle as seen from the base end side according to the third arrangement.
[Figure 25] Fig. 25 is a perspective view of a needle base of the injection needle as seen from the top end side according to the third arrangement.
[Figure 26] Fig. 26 is a perspective view of the needle base of the injection needle as seen from the base end side according to the third arrangement.
[Figure 27] Fig. 27 are views according to the third arrangement, in which, Fig. 27 (a) is a perspective view of a cap as seen from the top end side, Fig. 27 (b) is a perspective view of the cap as seen from the base end side, and Fig. 27 (c) is a longitudinal sectional view of the injection needle with the cap.
[Figure 28] Fig. 28 are views according to the third arrangement, in which, Fig. 28 (a) is perspective view of the injection needle with the cap as seen from the top end side, Fig. 28 (b) is a perspective view of the injection needle with the cap as seen from the base end side, and Fig. 28 (c) is a longitudinal sectional view of the injection needle with the cap.
[Figure 29] Fig. 29 are views according to a fourth arrangement, in which, Fig. 29 (a) is a perspective view of a cap, Fig. 29 (b) is a perspective view showing a state that a needle-base-side fitting part of the cap is fitted in the needle base. and Fig. 29 (c) is a perspective view showing a state that the cap is turned reversely so that an adjuster-side fitting part of the cap is fitted in the adjuster.

### MODE(S) FOR CARRYING OUT THE INVENTION

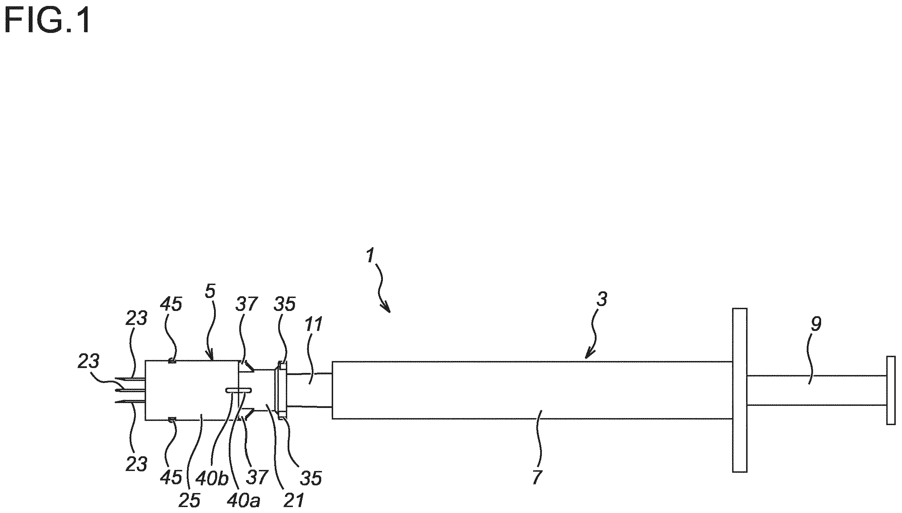

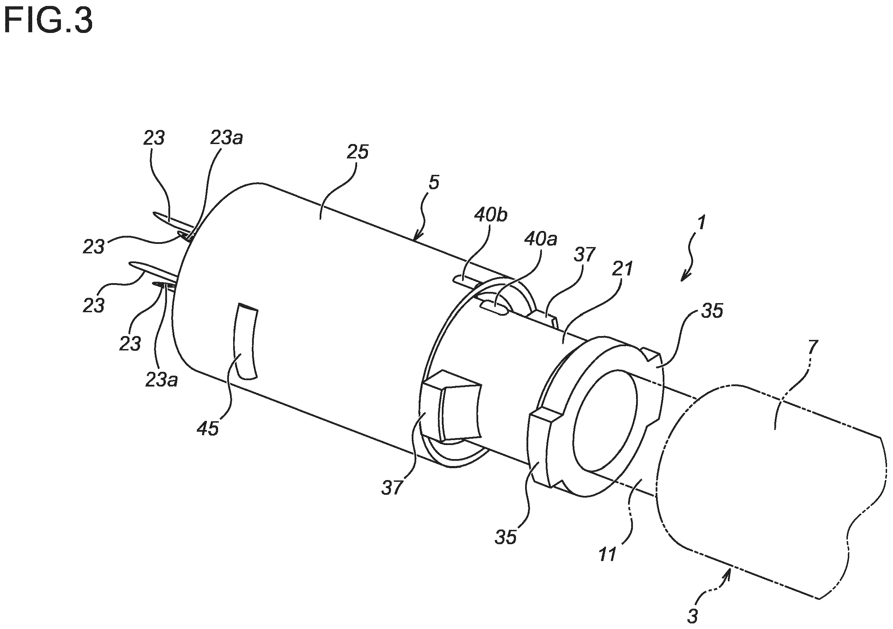

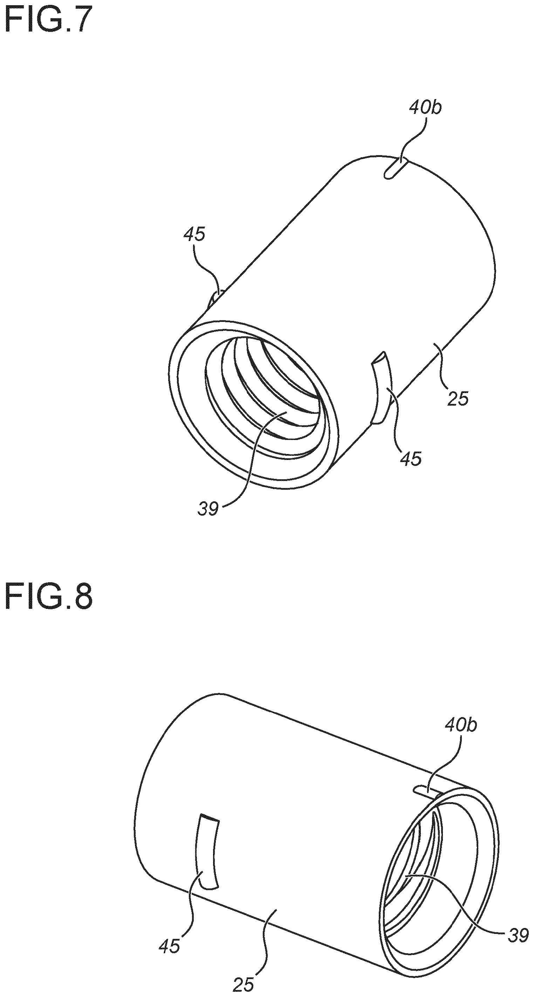

Now, a first arrangement will be explained with reference to Fig. 1 to Fig. 12. As illustrated in Fig. 1, an injection device 1 according to the first arrangement is composed of a syringe 3, and an injection needle 5 attached to be detachable to the top end of the syringe 3.

The syringe 3 is composed of an injection cylinder 7 in a hollow shape, of which top end side (on the left of Fig. 1) and base end side (on the right of Fig. 1) are opening, and a plunger 9 inserted into the injection cylinder 7 from the base end side (on the right of Fig. 1). The diameter at the top end side (on the left of Fig. 1) of the injection cylinder 7 is reduced, serving as an injection needle connecting part 11. The plunger 9 is disposed to be movable in the forward and rearward directions (in the left and right directions of Fig. 1) in the inside of the injection cylinder 7. The injection cylinder 7 is filled with an unillustrated medicinal solution therein, and by moving the plunger 9 to the top end side (to the left of Fig. 1), the medicinal solution is discharged from the top end of the injection cylinder 7.

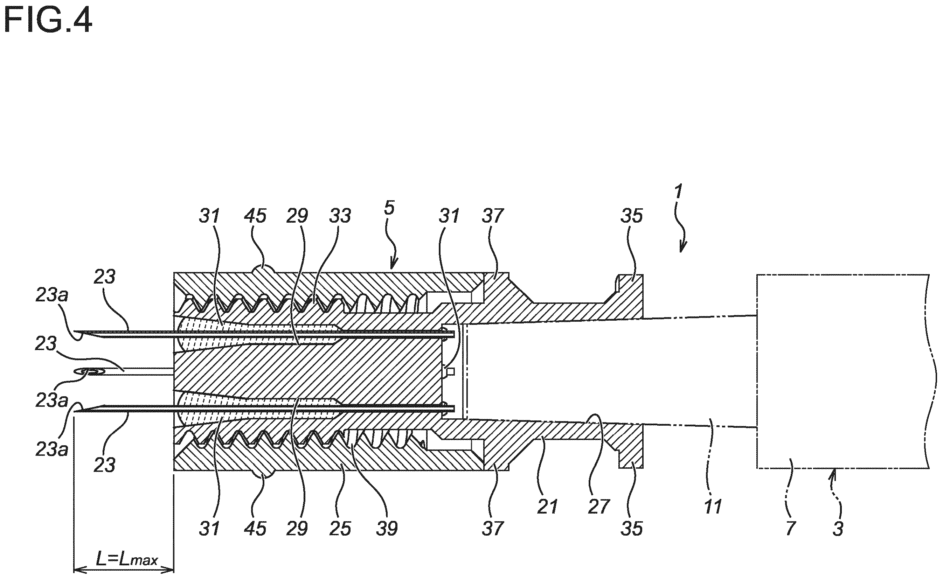

As illustrated in Fig. 1 to Fig. 4, the injection needle 5 is composed of a needle base 21, and four needles 23, 23, 23, 23, each of which being attached protrusively to the top end side (on the left of Fig. 4) of the needle base 21, and an adjuster 25.

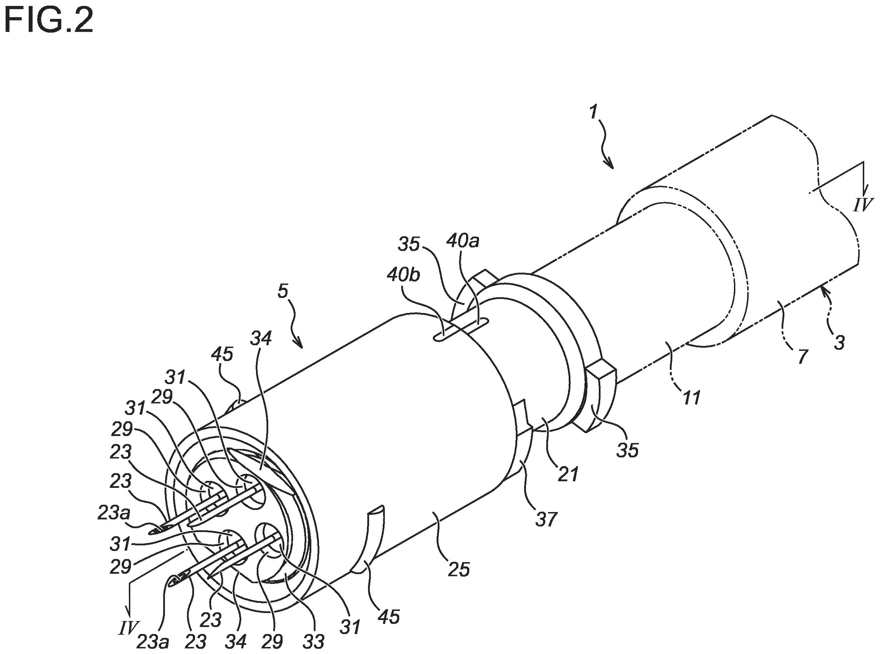

As illustrated in Fig. 4, a syringe connecting recess 27 is formed on the base end side of the needle base 21. The injection needle connecting part 11 of the syringe 3 is inserted into the syringe connecting recess 27. Moreover, as illustrated in Fig. 2, Fig. 4 and Fig. 5, four needle fixing penetration holes 29, 29, 29, 29, each of which has openings both on the side of the syringe connecting recess 27 and on the top end side (on the left of Fig. 4), are formed in the needle base 21. Each of the needles 23, 23, 23, 23 is penetrating through and disposed in the needle fixing penetration holes 29, 29, 29, 29, respectively, and is fixed by adhesives 31, 31, 31, 31, each of which being injected into the needle fixing penetration holes 29, 29, 29, 29, respectively. Moreover, a medicinal solution outlet 23a of each of the needles 23, 23, 23, 23 is cut obliquely, and is oriented inwardly in the radial direction of the needle base 21.

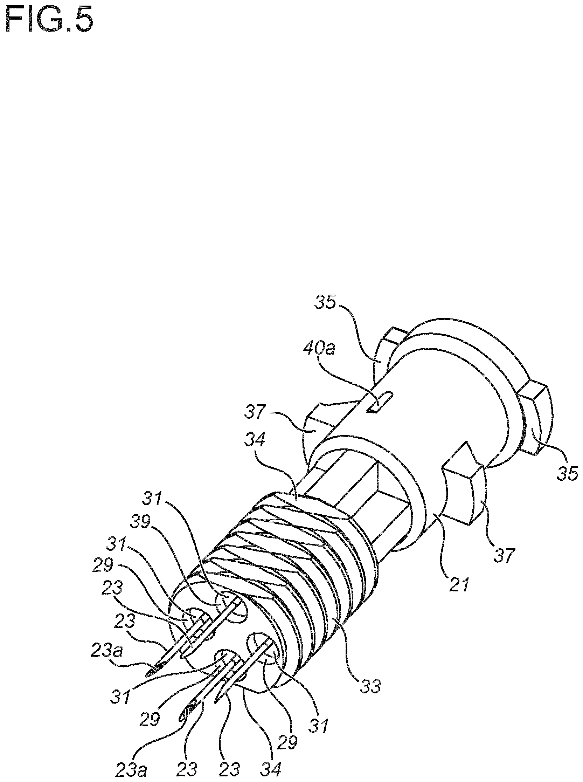

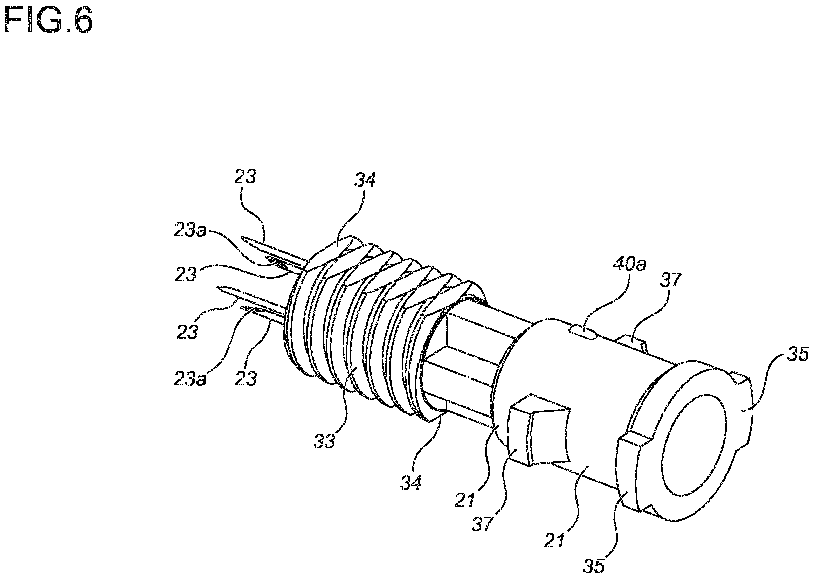

Moreover, as illustrated in Fig. 4, Fig. 5 and Fig. 6, a male screw portion 33 is formed on the top end side (on the left of Fig. 4) of the needle base 21. Moreover, as illustrated in Fig. 5 and Fig. 6, the top and bottom ends of the male screw portion 33, as shown in Fig. 5, have been cut off due to molding reasons. These cut-off portions are shown by reference signs 34, 34 in the drawings. Moreover, as illustrated in Fig. 4, Fig. 5 and Fig. 6, there are projections 35, 35, protrusively formed, respectively, at positions opposing to each other at the angle of 180 degrees. Moreover, at the position somewhat closer to the rear (to the right of Fig. 4) than the center of the axis direction (the left/right direction of Fig. 4) of the needle base 21, there are base-end side stoppers 37, 37, protrusively formed, respectively, at positions opposing to each other at the angle of 180 degrees.

As illustrated in Fig. 4, Fig. 7 and Fig. 8, the adjuster 25 is a member in a cylindrical shape, and a female thread portion 39 is formed in the inner peripheral surface thereof, to become in screw engagement with the male screw portion 33 of the needle base 21. The adjuster 25 is attached to the needle base 21 to be movable along the axis direction, by the screw engagement structure between the female thread portion 39 and the male screw portion 33 of the needle base 21.

With the rotative operation of the adjuster 25 so as to move along the axis direction (the left/right direction of Fig. 4) of the needle base 21, as illustrated in Fig. 4, Fig. 9 and Fig. 10, the protrusion length of the needles 23, 23, 23, 23 (as shown by reference sign L in Fig. 4), respectively, and eventually the puncture depth of the needles 23, 23, 23, 23, respectively, is adjusted. When the adjuster 25 is positioned at the right end of Fig. 4 so that the base end thereof becomes in contact with the base-end side stoppers 37, 37, the protrusion length (L) of the needles 23, 23, 23, 23, respectively, is in the maximum length (Lₘₐₓ).

For reference, in the present arrangement, the maximum protrusion length (Lₘₐₓ) of the needle 23, as shown in Fig. 4, is set to 4 mm, as an example.

From the above state, when the adjuster 25 is rotated counterclockwise as seen from the side facing to the direct front of the adjuster 25 at the left end in Fig. 4, so as to move to the left of Fig. 4, the needles 23, 23, 23, 23, respectively, are retracted gradually in the adjuster 25, and the protrusion length (L) of each of the needles 23, 23, 23, 23 becomes shorter gradually.

For reference, in the case of actual use, for the purpose of effective puncturing, the protrusion length (L) of each of the needles 23, 23, 23, 23 is set, for example, to be at least about 1 mm.

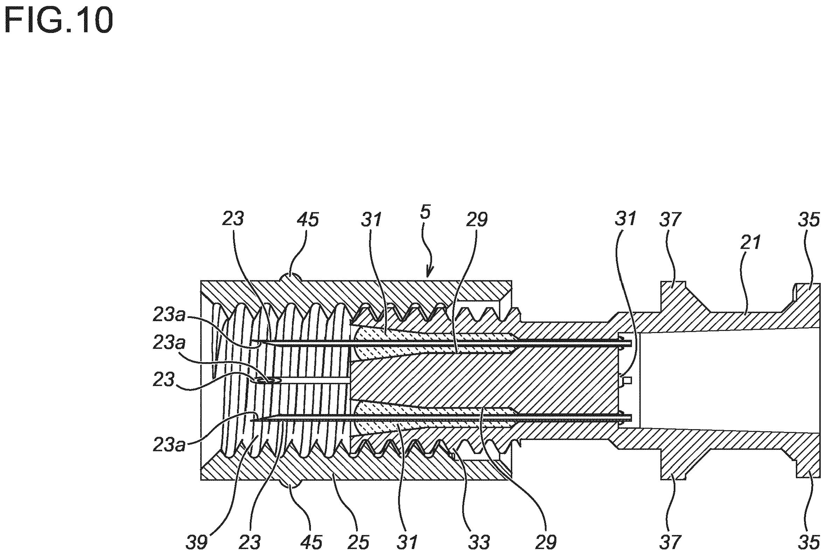

Moreover, after completion of puncture, as illustrated in Fig. 10, the adjuster 25 is moved to the top end side, until each of the needles 23, 23, 23, 23 is completely retracted in the inside of the adjuster 25. Accordingly, it is possible to prevent accidental needle pricking caused by double-puncture (wrong puncture after completion of puncture) by users such as physicians or nurses.

For reference, in Fig. 10, the top end of each of the needles 23, 23, 23, 23 is in a state of being withdrawn from the top end of the adjuster 25, for example, at least about 1 to 3 mm inwardly.

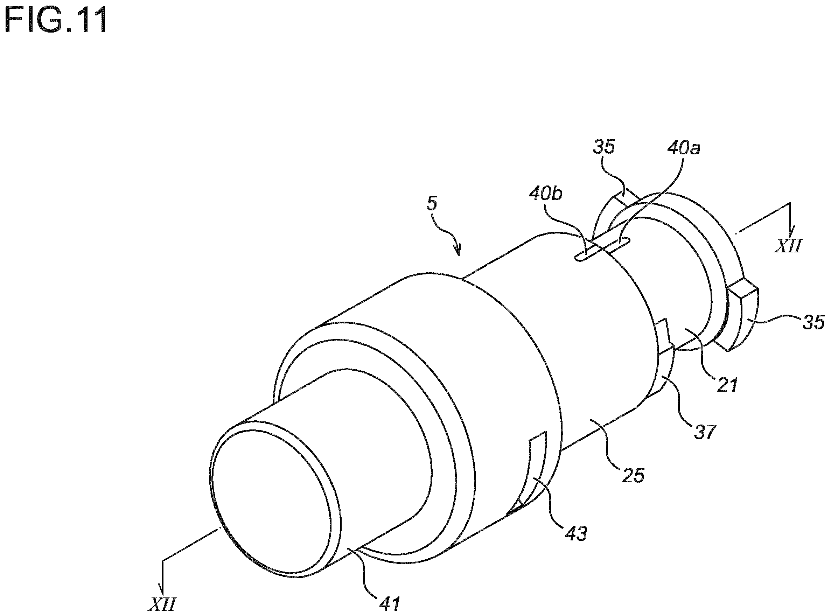

Moreover, as illustrated in Fig. 11, there is a needle-base side reference position marker 40a protrusively formed on the needle base 21, and there is also an adjuster side reference position marker 40b protrusively formed on the adjuster 25. The position in the rotative direction of the needle-base side reference position marker 40a and the position in the rotative direction of the adjuster side reference position marker 40b are set to match with each other, when the adjuster 25 is at the position closest to the base end side (a state as shown in Fig. 4).

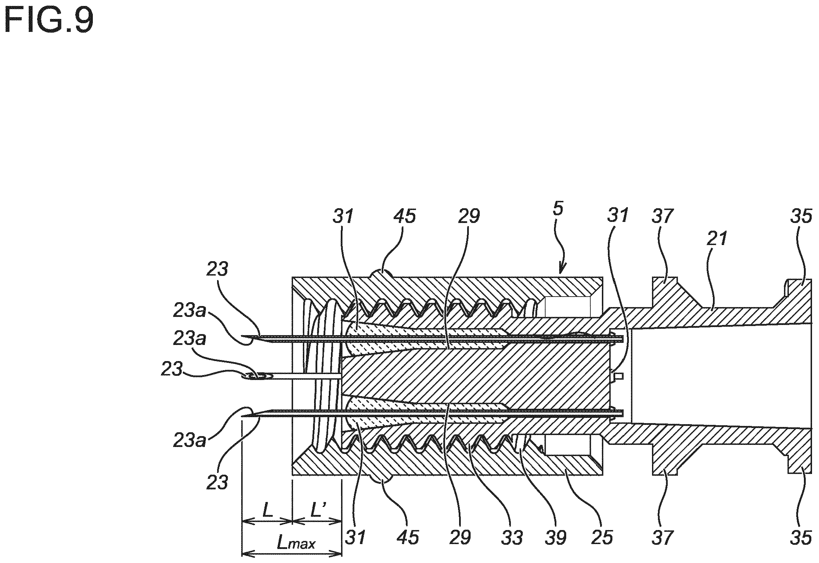

For example, when the adjuster 25 moves from an initial state as described above, toward the top end side, by referring to the needle-base side reference position marker 40a and also to the adjuster side reference position marker 40b, it is possible to visually recognize how many times the adjuster 25 was rotated. By referring to such a number of rotations and to the leading amount of the screw engagement structure between the male screw portion 33 and the female thread portion 39, it is possible to set the protrusion amount of each of the needles 23, 23, 23, 23 at an arbitrary value. This is to mean that, the maximum protrusion length (Lₘₐₓ) of each of the needles 23, 23, 23, 23 in the initial state of Fig. 4 has been determined (in the present arrangement, 4 mm), and meanwhile, with reference to the number of rotations of the adjuster 25, the moving amount of the adjuster 25 toward the top end side has also been determined. Thus, by deducting the moving amount (as shown by reference sign L' in Fig. 9) from the maximum protrusion length in the initial state (Lₘₐₓ), it is possible to set the protrusion length (L), and eventually the puncture depth, of each of the needles 23, 23, 23, 23.

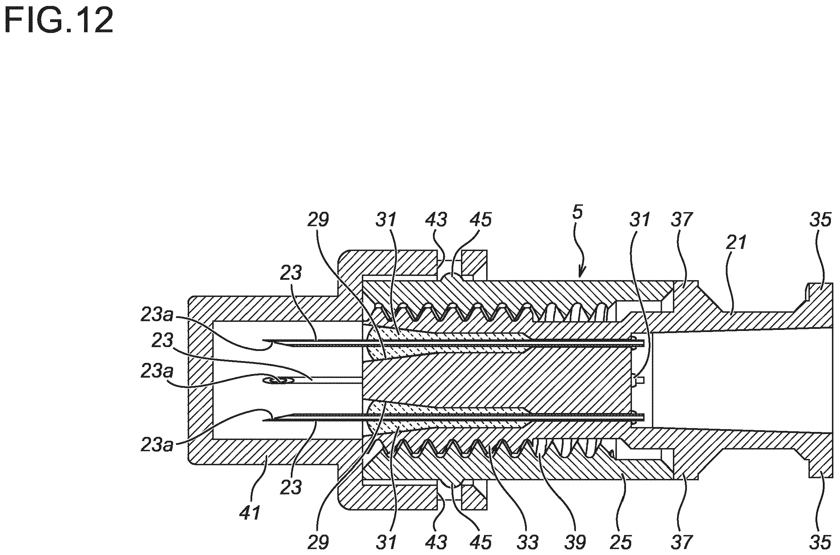

Moreover, as illustrated in Fig. 11 and Fig. 12, before using, a cap 41 is crowned to be detachable on the top end side (on the left of Fig. 12) of the adjuster 25 of the injection needle 5. There are engagement openings 43, 43, formed in the cap 41, respectively, at positions opposing to each other at the angle of 180 degrees, and there are also engagement projections 45, 45, protrusively formed, respectively, at positions opposing to each other at the angle of 180 degrees, on the outer periphery of the adjuster 25. By engagement of the engagement openings 43, 43 of the cap 41 with the engagement projections 45, 45 of the adjuster 25, respectively, the cap 41 is fixed in the adjuster 25.

For reference, as illustrated in Fig. 12, even in the initial state in which each of the needles 23, 23, 23, 23 is protruding toward the top end side (the left side of Fig. 12) of the adjuster 25, the top end of each of the needles 23, 23, 23, 23 will not become in contact with the inner surface of the cap 41.

Next, the function according to the first arrangement will be explained.

First, before using, the injection needle 5 is in a state as shown in Fig. 11 and Fig. 12.

Next, the medicinal solution is filled in the injection cylinder 7 of the syringe 3.

Next, the injection needle 5 is attached to the injection cylinder 7 of the syringe 3.

Next, the cap 41 is detached, so as to be in a usable state as illustrated in Fig. 1 to Fig. 4. In such a state, the protrusion length (L) of each of the needles 23, 23, 23, 23 is at the maximum protrusion length (Lₘₐₓ).

Next, the adjuster 25 is rotated counterclockwise as seen from the side facing to the direct front of the adjuster 25 at the left end in Fig. 1 to Fig. 4, so as to move the adjuster 25 to the left of Fig. 1 to Fig. 4 at an appropriate amount, whereby the protrusion length (L) of each of the needles 23, 23, 23, 23, and eventually the puncture depth of each of the needles 23, 23, 23, 23, is adjusted arbitrarily.

Next, the side of the needles 23, 23, 23, 23 of the injection device 1 is oriented to the skin (not shown) as an object to be punctured, and the needles 23, 23, 23, 23 are punctured vertically against the skin. At that time, the top end surface of the adjuster 25 (the surface on the left of Fig. 1 to Fig. 4) becomes in contact with the skin, and the puncture is performed at a predetermined puncture depth.

Next, the plunger 9 of the syringe 3 is depressed to apply force toward the top end side (the left of Fig. 1 to Fig. 4), whereby the medicinal solution is injected.

After completion of injection, the needles 23, 23, 23, 23 are removed from the skin. Thereafter, the adjuster 25 is rotated counterclockwise as seen from the side facing to the direct front of the adjuster 25 at the left end in Fig. 1 to Fig. 4, so as to move the adjuster 25 to the top end side (to the left of Fig. 1 to Fig. 4), and as illustrated in Fig. 10, the top end side of each of the needles 23, 23, 23, 23 is retracted in the adjuster 25. Thus, the double-puncture (wrong puncture after completion of puncture) is prevented. Further, the top end is covered by crowning of the cap 41, and the whole body of the injection device 1 is disposed as the waste in such a state. Otherwise, only the injection needle 5 is disposed as the waste, by detaching the injection needle 5 from the syringe 3.

Next, the effect according to the first arrangement will be explained.

First, it is possible to arbitrarily adjust the protrusion length (L), and eventually the puncture depth of each of the needles 23, 23, 23, 23. This is because of the structure in which the adjuster 25 is attached to be movable to the outer periphery of the needle base 21, so that the position in the axis direction can be adjusted arbitrarily.

Moreover, since the protrusion length (L), and eventually the puncture depth of each of the needles 23, 23, 23, 23 can be adjusted arbitrarily, it is not necessary to prepare several types of injection needles 5, each of which protrusion length is different from each other, and accordingly, in addition to the reduction of cost, it is also possible to accomplish the easy management of the injection needles 5.

Moreover, as illustrated in Fig. 10, after completion of puncture, the top end of each of the needles 23, 23, 23, 23 can be retracted in the adjuster 25, and accordingly, even without the need of attachment of the cap 41, it is possible to prevent accidental needle pricking caused by double-puncture (wrong puncture after completion of puncture) by users such as physicians or nurses.

Moreover, with the attachment of the cap 41, the safety can be further improved.

Moreover, before using, the cap 41 is crowned on the top end side of the adjuster 25, and accordingly, for example, it is possible to prevent injury of a user before using.

Moreover, since the adjuster 25 is attached to be movable on the outer periphery of the needle base 21 by the screw engagement structure, it is possible to move the adjuster 25 easily, and accordingly, the adjustment of the protrusion length (L), and eventually the puncture depth of each of the needles 23, 23, 23, 23, can be facilitated.

Moreover, the needle-base side reference position marker 40a is formed on the needle base 21, and the adjuster side reference position marker 40b is also formed on the adjuster 25, and accordingly, it is possible to visually recognize the number of rotations of the adjuster 25, whereby the protrusion length (L), and eventually the puncture depth of each of the needles 23, 23, 23, 23, can be adjusted accurately.

And moreover, since the medicinal solution outlet 23a of each of the needles 23, 23, 23, 23 is oriented inwardly in the radial direction of the needle base 21, the injected medicinal solution will diffuse widely, and simultaneously, will remain in a shallow portion of the skin.

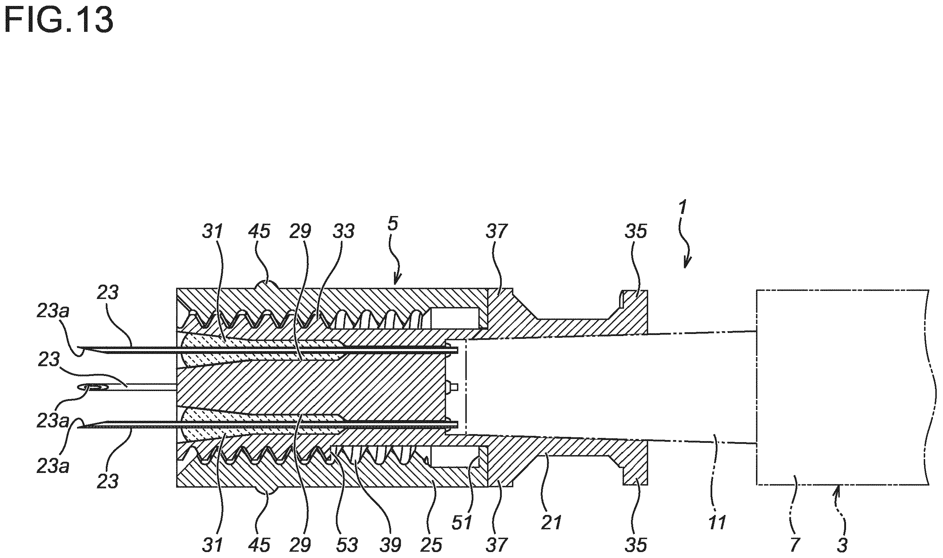

Next, a second arrangement will be explained with reference to Fig. 13 to Fig. 15. In the first arrangement, the moving amount of the adjuster 25 toward the top end side is not limited specifically. However, in the second arrangement, this amount is limited to a predetermined value.

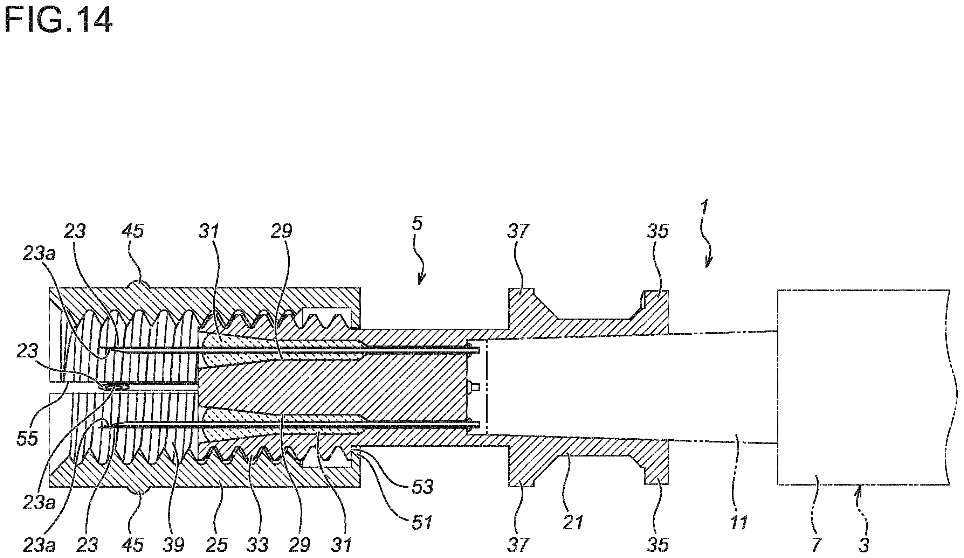

First, as illustrated in Fig. 13, an adjuster-side stopper 51 is provided on the base end side (on the left end side of Fig. 13) of the adjuster 25. The adjuster-side stopper 51 is in a ring shape, and is provided in a shape of collar. Meanwhile, the base end side (the right end side of Fig. 13) of the female thread portion 33, attached to the needle base 21, serves as a needle-base-side stopper 53. Thus, with the movement of the adjuster 25 to the top end side (to the left of Fig. 13), and as illustrated in Fig. 14, when the adjuster-side stopper 51 becomes in contact with the needle-base-side stopper 53, the further movement of the adjuster 25 toward the top end side (the left of Fig. 14) is prohibited. Accordingly, the needles 23, 23, 23, 23 are retracted securely in the adjuster 25, and also the drop-off of the adjuster 25 from the needle base 21 is prevented.

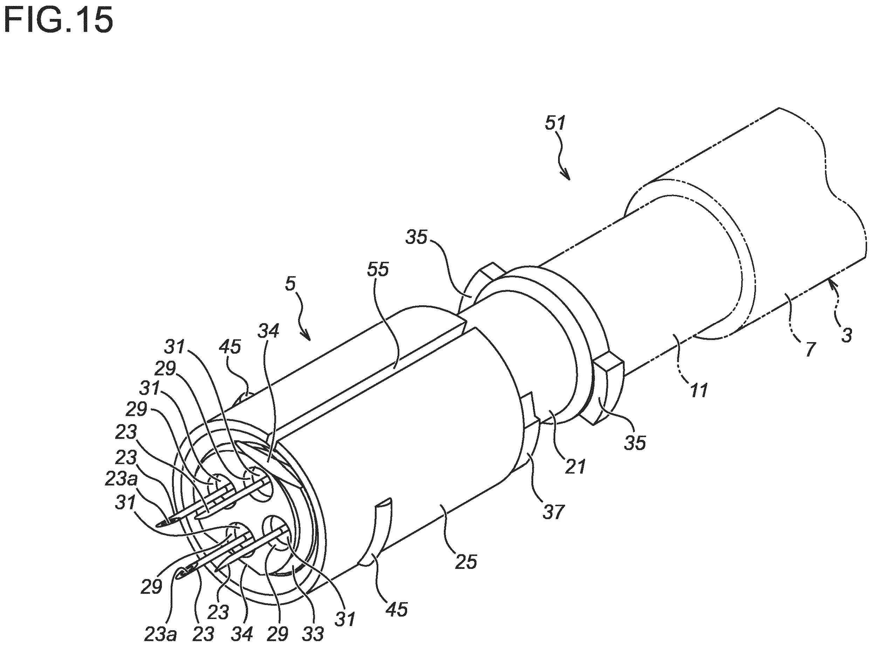

Moreover, since the adjuster-side stopper 51 is provided to the adjuster 25, there is a concern about the attachment of the adjuster 25 to the needle base 21. To cope with this concern, as illustrated in Fig. 15, a longitudinally-split groove 55 is provided in the adjuster 25. When the adjuster 25 is attached to the needle base 21, the attachment is performed by expanding the diameter of the adjuster 25, to some width within the range of the longitudinally-split groove 55.

The other structure is substantially the same as that of the first arrangement, and the same reference signs will be allotted to the same elements in the drawings, and the explain thereof will be omitted.

According to the above structure, it is possible to achieve substantially the same effect as that of the first arrangement explained above.

Moreover, since the moving amount of the adjuster 25 to the top end side is limited to a predetermined value, it is possible to prevent any unintended drop-off of the adjuster 25 from the needle base 21.

Moreover, when a user moves forward the adjuster 25 until the adjuster-side stopper 51 becomes in contact with the needle-base-side stopper 53, the user is able to confirm that the needles 23, 23, 23, 23 are retracted in the adjuster 25, at the position capable of preventing the double-puncture (wrong puncture after completion of puncture).

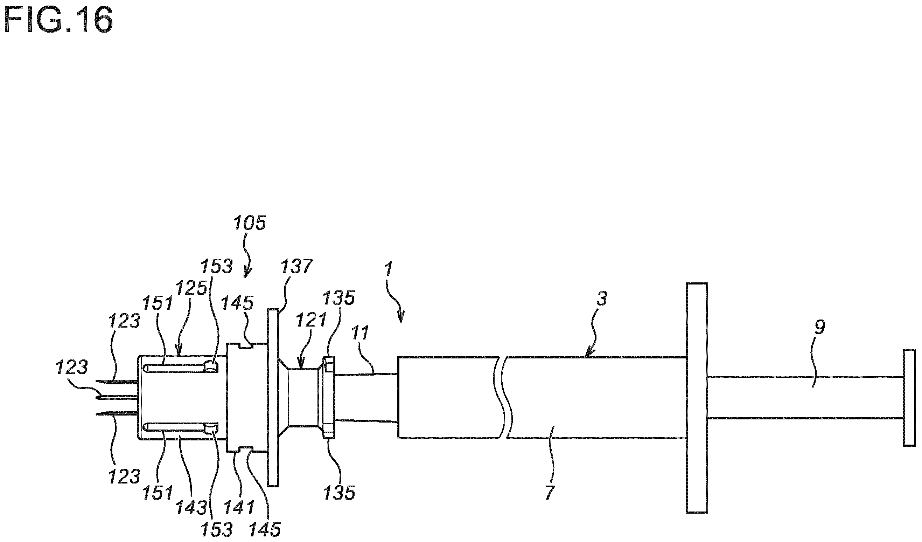

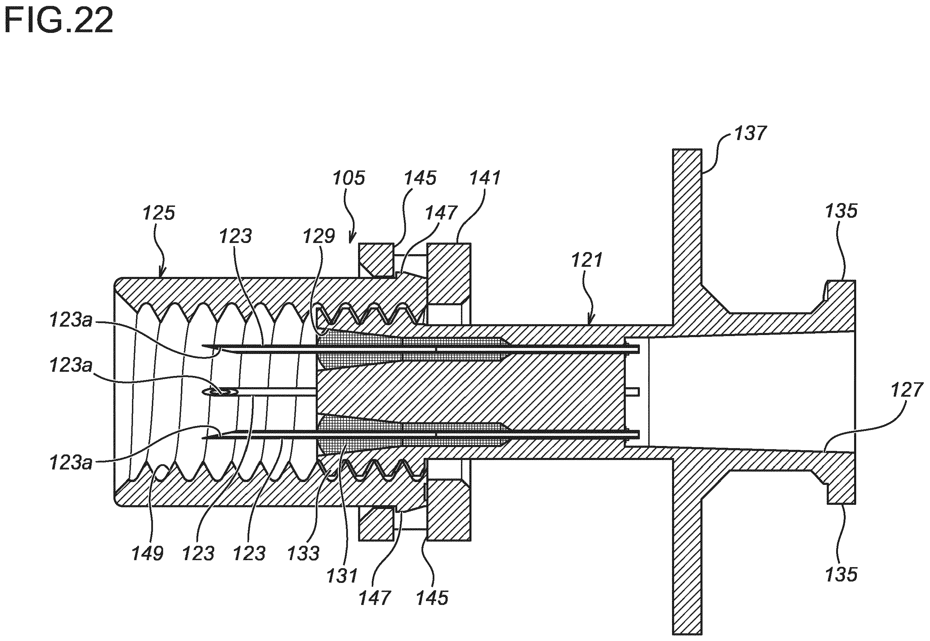

Next, a third arrangement will be explained with reference to Fig. 16 to Fig. 28. As illustrated in Fig. 16 to Fig. 22, an injection needle 105 according to the third arrangement is composed of a needle base 121, and four needles 123, 123, 123, 123, each of which being attached protrusively to the top end side (on the left of Fig. 16 of the needle base 121, and an adjuster 125.

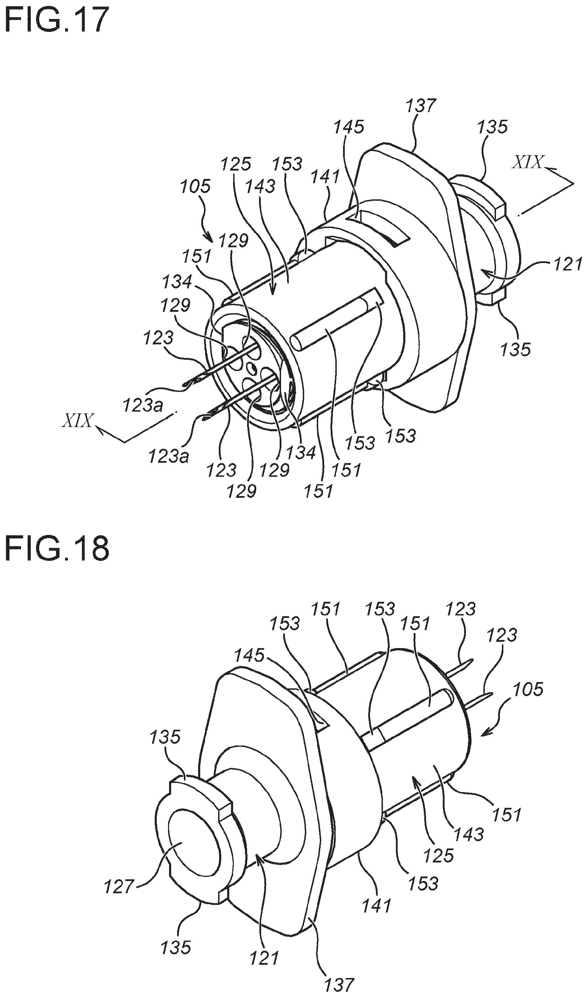

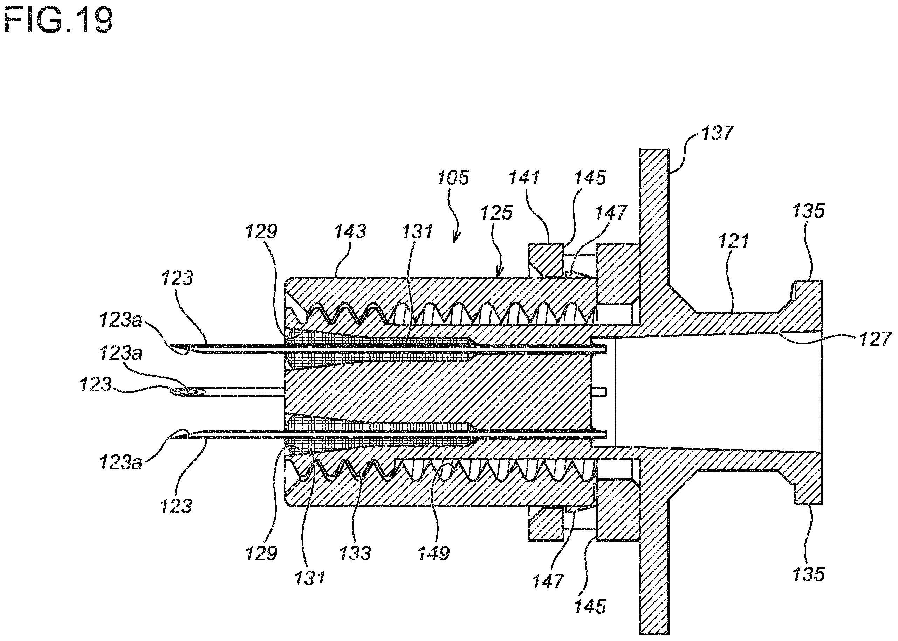

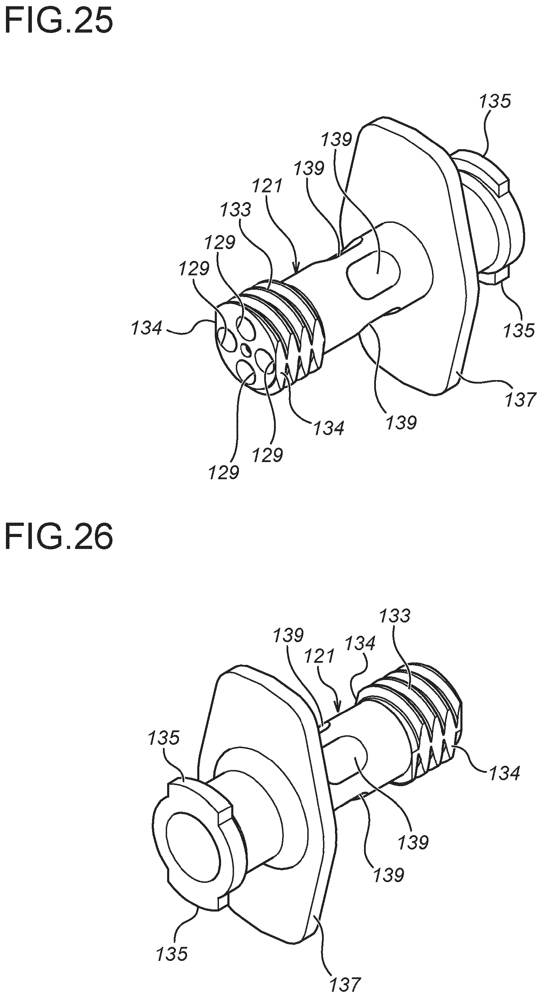

As illustrated in Fig. 19, a syringe connecting recess 127 is formed on the base end side of the needle base 121. The injection needle connecting part 11 of the syringe 3 is inserted into the syringe connecting recess 127. Moreover, as illustrated in Fig. 17 and Fig. 19, four needle fixing penetration holes 129, 129, 129, 129, each of which has openings both on the side of the syringe connecting recess 127 and on the top end side (on the left of Fig. 25), are formed in the needle base 121.

Each of the needles 123, 123, 123, 123 is penetrating through and disposed in the needle fixing penetration holes 129, 129, 129, 129, respectively, and is fixed by adhesives 131, 131, 131, 131, each of which being injected into the needle fixing penetration holes 129, 129, 129, 129, respectively. Moreover, as illustrated in Fig. 17 and Fig. 19, a medicinal solution outlet 123a of each of the needles 123, 123, 123, 123 is cut obliquely, and is oriented inwardly in the radial direction of the needle base 121.

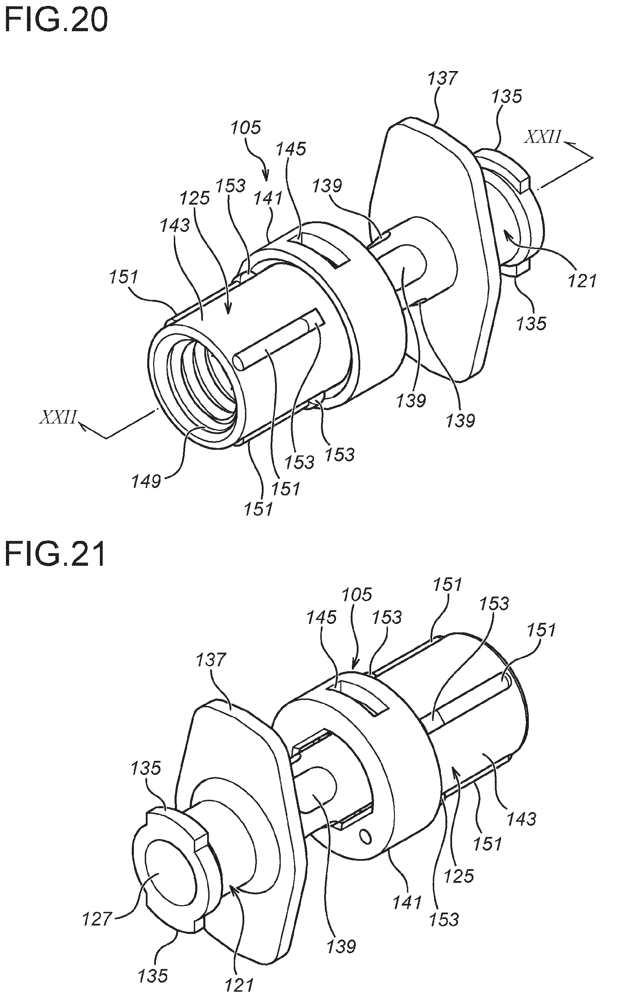

Moreover, as illustrated in Fig. 19, a male screw portion 133 is formed on the top end side (on the left of Fig. 19) of the needle base 121. Moreover, as illustrated in Fig. 25 and Fig. 26, the both transverse side portions of the male screw portion 133 have been cut off, serving as cut-off portions 134, 134. Moreover, as illustrated in Fig. 19, Fig. 20 and Fig. 21, there are projections 135, 135, protrusively formed, respectively, at positions opposing to each other at the angle of 180 degrees. Moreover, at the position somewhat closer to the rear (to the right of Fig. 19) than the center of the axis direction (the left/right direction of Fig. 19) of the needle base 121, there is a substantially-diamond-shaped holding projection 137, formed protrusively in a shape of collar. The holding projection 137 also has a function to serve as a stopper to limit the rearward movement of the adjuster 125 to the base end side, to a predetermined amount. Moreover, as illustrated in Fig. 20, Fig. 21, Fig. 25 and Fig. 26, the needle base 121 is provided with four recesses 139, 139, 139, 139, each of which being disposed orthogonally, for the purpose of prevention of sink (depression due to shrinking) during molding work.

As illustrated in Fig. 17, Fig. 18 and Fig. 19, the adjuster 125 is composed of a collar portion 141, and a cylindrical portion 143 fastened to the collar portion 141. There are openings 145, 145, formed in the cylindrical surface of the collar portion 141, respectively, at positions opposing to each other at the angle of 180 degrees. Moreover, there are claws 147, 147, protrusively disposed at positions opposing to each other at the angle of 180 degrees, respectively, at the end part of the cylindrical portion 143 on the side of the collar portion 141. When the cylindrical portion 143 is inserted into the inner diametrical portion of the collar portion 141, the claws 147, 147 become engaged with the openings 145, 145, respectively, whereby the cylindrical portion 143 is integrated into the collar portion 141.

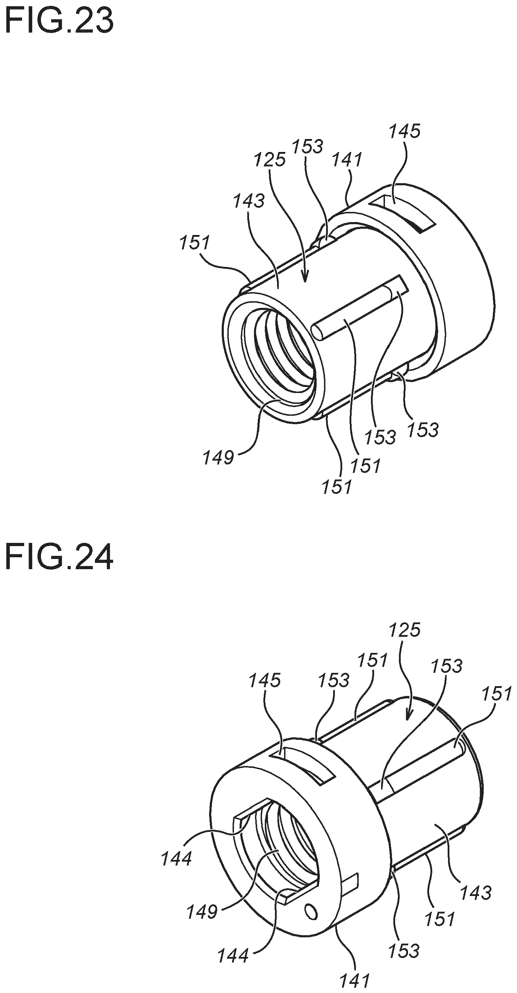

Moreover, as illustrated in Fig. 24, an opening 142 of the collar portion 141 is in a circular shape, of which two transverse side portions have been cut away, so as to form flat surfaces 144, 144, respectively. This shape corresponds to the shape of the male screw portion 133 of the needle base 121. When the male screw portion 133 of the needle base 121 penetrates through the inside of the adjuster 125, the positions of the cut-off portions 134, 134 of the male screw portion 133 of the needle base 121, and the positions of the flat surfaces 144, 144 of the opening 142 of the collar portion 141 of the adjuster 125, shall be matched to each other. Moreover, after completion of attachment, when the adjuster 125 is rotated so as to move to the top end side, the collar portion 141 becomes in contact with the end of the male screw portion 133, whereby the stopper function is exhibited, and further movement is prohibited. When the position of the cut-off portions 134, 134 of the male screw portion 133 of the needle base 121, and the positions of the flat surfaces 144, 144 of the opening 142 of the collar portion 141 of the adjuster 125, are matched to each other, the pulling off thereof can be performed, but the movement with the rotation cannot be performed.

There is a female thread portion 149 formed in the inner peripheral surface of the cylindrical surface 143, to become in screw engagement with the male screw portion 133 of the needle base 121. The adjuster 125 is attached to the needle base 121 to be movable along the axis direction, by the screw engagement structure between the female thread portion 149 and the male screw portion 133 of the needle base 121.

As illustrated in Fig. 16 to Fig. 18, Fig. 20 and Fig. 21, there are rib-shaped guide projections 151 151, 151, 151, respectively, protrusively provided at four orthogonal positions on the outer peripheral surface of the cylindrical portion 143. Moreover, there are engagement projections 153, 153, 153, 153, protrusively provided at the end part of the guide projections 151, 151, 151, 151, respectively, on the side of the collar portion 141.

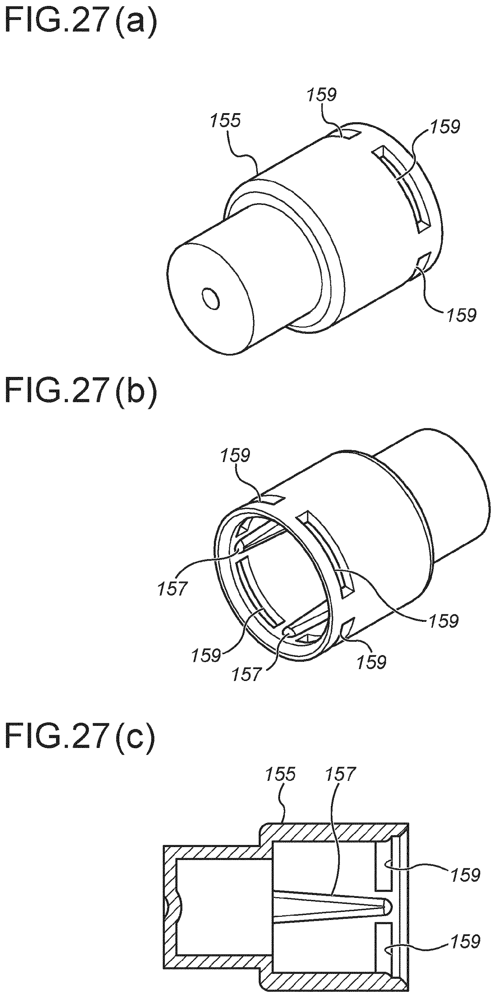

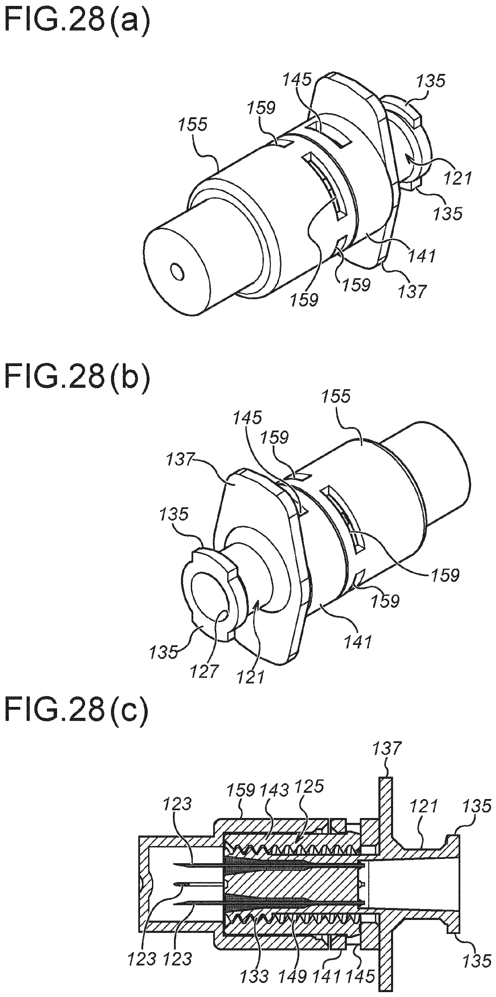

Meanwhile, as illustrated in Fig. 27 and Fig. 28, there is a cap 155, and guide projections 157, 157, 157, 157 are provided, respectively, on the inner peripheral surface of the cap 155, to be engaged with the guide projections 151, 151, 151, 151, respectively, in the rotative direction. This is to mean that, when the cap 155 is crowned on the adjuster 125, the guide projections 157, 157, 157, 157, on the side of the cap 155, are positioned, in a respective interval of the guide projections 151, 151, 151, 151, on the side of the adjuster 125. Moreover, each of the guide projections 157, 157, 157, 157, is provided in a form of V-shape elongating in the longitudinal direction. Consequently, the guide function is exhibited effectively, and the crowning of the cap 155 on the adjuster 125 can be performed smoothly. Moreover, there are engagement openings 159, 159, 159, 159, respectively provided in the intervals of the guide projections 157, 157, 157, 157. Each of the engagement openings 159, 159, 159, 159, is engaged with the respective engagement projections 153, 153, 153, 153.

Fig. 28 shows a state, in which the cap 155 is crowned on the injection needle 105.

The other structure is substantially the same as that of the first arrangement, and the same reference signs will be allotted to the same elements in the drawings, and the explain thereof will be omitted.

Accordingly, it is possible to achieve substantially the same effect as that of the first arrangement described above.

Moreover, since the holding projection 137 is provided, for example, when the injection needle 105 is attached to the top of the syringe 3, the holding projection 137 is held by the left hand, and at the same time, the syringe 3 is held by the right hand, whereby the injection needle connecting part 11 can be inserted into the syringe connecting recess 127 so as to be attached thereto, and consequently, it is not necessary to hold the cap 155 or the adjuster 125, and there is no risk of unintended rotation of the adjuster 125 to fasten tightly.

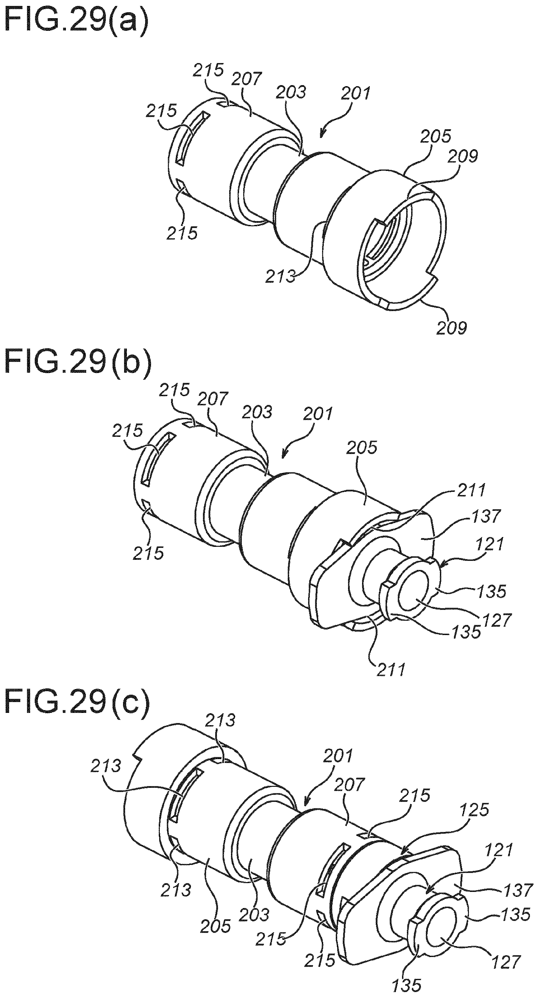

Next, a fourth arrangement will be explained with reference to Fig. 29. In the case of the first to the third arrangements, the cap is fitted in the adjuster of the injection needle in a state that the rotation thereof is prohibited. On the other hand, in the case of the fourth arrangement, the cap is fitted in the needle base side in a state that the rotation thereof is prohibited. The details will be explained hereinafter.

As illustrated in Fig. 29 (a), a cap 201 is provided with a cap body 203, and a needle-base-side fitting portion 205 is provided on one end side of the cap body 203, and furthermore, an adjuster-side fitting portion 207 is provided on the other end side of the cap body 203. There are engagement portions 209, 209, provided respectively on the needle-base-side fitting portion 205, at positions opposing to each other at the angle of 180 degrees. As illustrated in Fig. 29 (b), the engagement portions 209, 209 become engaged with engagement portions 211, 211, respectively, of the holding projection 137 of the needle base 121.

There are four engagement opening 213, 213, 213, 213, respectively formed at orthogonal positions to each other, in the needle-base-side fitting portion as described above. The engagement openings 213, 213, 213, 213 become engaged with four engagement projections (the engagement projections, having substantially the same structure as that of the engagement projections 153 of the third arrangement), respectively, provided on the adjuster 125.

There are also four engagement opening 215, 215, 215, 215, respectively formed at orthogonal positions to each other, in the adjuster-side fitting portion 207. The engagement openings 215, 215, 215, 215 also become engaged with the four engagement projections as described above, respectively, provided on the adjuster 125.

The other structure is substantially the same as that of the third arrangement, and the same reference signs will be allotted to the same elements in the drawings, and the explain thereof will be omitted.

According to the above structure, the normal state is shown in Fig. 29 (b), in which, the needle-base-side fitting portion 205 of the cap 201, enclosing the adjuster 125 of the injection needle, has been fitted in a part of the needle base 121, and also in which, the engagement portions 209, 209 of the needle-base-side fitting portion 205 are engaged with the engagement portions 211, 211, respectively, of the holding projection 137 of the needle base 121. In this state, when the injection needle 105 is attached to the top of the syringe, the cap 201 is held by the left hand, and at the same time, the syringe is held by the right hand, whereby the injection needle connecting part of the syringe is inserted into the syringe connecting recess 127. At that time, since the cap 201 is fixed in the holding projection 137 and eventually in the needle base 121, there is no risk of unintended rotation of the cap 201, and consequently, there is also no risk of unintended rotation of the adjuster 125.

Next, the adjustment of the protrusion length of the needle will be explained. For this purpose, as illustrated in Fig. 29 (c), the adjuster-side fitting portion 207 of the cap 201 becomes engaged with the adjuster 125. In such a state, by holding the cap 201, the adjuster 125 is rotated, so as to adjust the protrusion length of the needle. Consequently, it is possible to prevent accidental needle pricking at the time of adjustment of the protrusion length of the needle.

The present invention is not limited to the first to the fourth arrangement as described above.

First, in the first to the fourth arrangements, the explanations have been made for the structure of four needles as an example. However, it is of course possible to be composed of other structures, such as one needle, or two, three, five or more needles.

Moreover, in the first to the fourth arrangements, the adjuster is movable by the screw engagement structure. However, the present invention is not limited to such a structure, and it is also possible, for example, to be movable by an appropriate fit-in-each-other structure.

Moreover, in the second arrangement, it is possible to use the longitudinally-split groove also as the adjuster side reference position marker.

Moreover, in the second arrangement, with the stopper mechanism, the needle is securely retracted by the adjuster, and the drop-off of the adjuster is prevented. However, it is also possible to utilize in the different structure, for the purpose of limiting the protrusion length of the needle within a predetermined range. As a possible structure, for example, in the initial state before using, the protrusion length of the needle may be set to 4 mm, and when the forward movement of the adjuster is prohibited by the stopper mechanism, the protrusion length of the needle may be set to 1 mm.

Moreover, in the first to the fourth arrangements, the adjuster is rotated counterclockwise as seen from the side facing to the direct front of the adjuster, so as to shorten the protrusion length of the needle. However, it is also possible to rotate in the reverse direction.

Moreover, in the first to the fourth arrangements, the adjuster is in the hollow cylindrical shape. However, the present invention is not limited to such a structure, and it is possible, for example, to be in a square shape, or any other shape. For example, in the case of square shape, if one rotation varies the length by 1 mm, then, the rotation at the angle of 90 degrees may vary the protrusion length by 1/4 mm.

Moreover, in the first to the fourth arrangements, the adjuster is rotated in a state that the cap has been detached, so as to adjust the protrusion length of the needle. However, it is also possible to make such an adjustment in a state that the cap remains in the crowned state, whereby the accidental needle pricking can also be prevented.

Moreover, it is also possible to provide scales of the protrusion length of the needle for reference purpose, on the adjuster or the needle base, by molding or printing.

Moreover, in the first to the fourth arrangements, the medicinal solution outlet of the needle is oriented inwardly in the radial direction, but the direction is not limited to these arrangements.

Moreover, in the fourth arrangement, the needle-base-side fitting portion and the adjuster-side fitting portion are provided in the opposite directions, respectively. However, it is also possible to provide the needle-base-side fitting portion only. In this structure, it is also possible that, by pulling the cap at a predetermined amount toward the top end side, the needle-base-side fitting portion is released from the fitting state in the needle base, and then, is fitted in the adjuster. Thus, in such a state, the protrusion length of the needle can also be adjusted by rotating the adjuster integrally with the cap.

For example, in the fourth arrangement, in the state as illustrated in Fig. 29 (b), the engagement openings 213 is engaged with the engagement projection on the side of the adjuster. However, with regard to the engagement opening 213, by increasing the length in the axis direction thereof, even when the cap is pulled toward the top end side at a predetermined amount so as to release the fitting of the needle-base-side fitting portion out of the needle base, the engagement opening 213 may maintain the engagement state with the engagement projection on the side of the adjuster, and thus, in such a state, the protrusion length of the needle can be adjusted by rotating the adjuster integrally with the cap.

Moreover, as an another example, in the state as illustrated in Fig. 29 (b) according to the fourth arrangement, it is also possible to provide a structure, in which, the engagement opening 213 passes by the engagement projection on the side of the adjuster without engagement therewith, and when the cap is pulled toward the top end side at a predetermined amount so as to release the fitting of the needle-base-side fitting portion out of the needle base, the engagement opening 213 becomes engaged with the engagement projection on the side of the adjuster. Thus, also in such a state, the protrusion length of the needle can be adjusted by rotating the adjuster integrally with the cap.

Furthermore, the structures shown in the drawings are merely for example purposes only.

### INDUSTRIAL APPLICABILITY

The present invention relates to the injection needle and the injection device for performing prevention and treatment of various diseases, for example by subcutaneous or intradermal administration of an objective substance, and more particularly, the present invention relates to the injection needle and the injection device, of which puncture depth of a needle can be adjusted. The present invention is suitable, for example, for the injection needle and the injection device used for treatment in the field of cosmetic dermatology.

### EXPLANATION OF REFERENCE NUMERALS AND SIGNS

- 1: Injection Device
- 3: Syringe
- 5: Injection Needle
- 21: Needle Base
- 23: Needle
- 23a: Medicinal Solution Outlet
- 25: Adjuster
- 33: Male Screw Portion
- 39: Female Thread Portion
- 40a: Needle-base Side Reference Position Marker
- 40b: Adjuster Side Reference Position Marker
- 41: Cap
- 51: Adjuster-side Stopper
- 53: Needle-base-side Stopper
- 55: Longitudinally-split Groove

## Claims

1. An injection needle provided with:
a needle (23, 123);
a needle base (21) holding the needle (23, 123) in a state of protruding toward the top end side; and an adjuster (25, 125) disposed to be movable along the axis direction of the needle base (21) on the outer periphery of the needle base (21), wherein a protrusion length of the needle (23, 123) is adjusted by adjusting a position of the adjuster (25, 125) in the axis direction;
the adjuster (25, 125) is movable in the axis direction of the needle base (21) by a screw engagement structure;
**characterised in that**
the moving amount of the adjuster (25, 125) toward the top end side is limited to a predetermined amount by a stopper mechanism (51, 53);
the stopper mechanism (51, 53) is composed of a collar portion (51) on the side of the adjuster (25, 125) becoming in contact with an end of a male screw portion (33) on the side of the needle base (21).

2. The injection needle as claimed in Claim 1, wherein, the top end of the needle (23, 123) is capable of being retracted in the adjuster (25, 125).

3. The injection needle as claimed in Claim 1, wherein, a needle base (21) is provided with a needle-base side reference position marker (40a), and the adjuster (25, 125) is provided with an adjuster side reference position marker (40b).

4. The injection needle as claimed in Claim 1, wherein,
the adjuster (25, 125) is provided with a longitudinally-split groove (55), so as to allow expansion of the diameter when attaching to the needle base (21).

5. The injection needle as claimed in Claim 1, wherein, the cross-section shape of the male screw portion (33) of the needle base (21) is in a non-circular shape, and the cross-section shape of an opening of the collar portion (51) of the adjuster is also in a non-circular shape correspondingly, and by matching each position in the rotative direction, the male screw portion (33) becomes capable of penetrating through the inside of the adjuster (25, 125).

6. The injection needle as claimed in any one Claim among Claim 1 to Claim 5, wherein, the needle (23, 123) is provided in a plural number.

7. The injection needle as claimed in Claim 6, wherein, a medicinal solution outlet (23, 123a) of the needle (23, 123) is oriented inwardly in the radial direction of the needle base (21).

8. The injection needle as claimed in any one Claim among Claim 1 to Claim 7, wherein, the needle base (21) is provided with a holding projection.

9. The injection needle as claimed in any one Claim among Claim 1 to Claim 8, wherein, the outer periphery of the adjuster (25, 125) is crowned with a cap (41, 201) so as to cover the needle.

10. The injection needle as claimed in Claim 9, wherein,
the cap (41) is fitted in the adjuster, so that the adjuster (25, 125) is capable of being rotated with the rotation of the cap (41).

11. The injection needle as claimed in Claim 10, wherein,
the adjuster (25, 125) is provided with an engagement projection (45), and the cap (41) is provided with an engagement opening to be engaged with the engagement projection (45).

12. The injection needle as claimed in Claim 9, wherein, the cap encloses the adjuster and is fitted in the needle base in a state that the rotation is prohibited.

## Patentansprüche

1. Injektionsnadel, die Folgendes umfasst:
eine Nadel (23, 123);
eine Nadelbasis (21), die die Nadel (23, 123) in einem Zustand hält, in dem sie zur Oberseite hin vorsteht; und einem Einsteller (25, 125), der entlang der Achsenrichtung der Nadelbasis (21) an der Außenumfangsfläche der Nadelbasis (21) bewegbar angeordnet ist, wobei eine vorstehende Länge der Nadel (23, 123) durch Einstellen einer Position des Einstellers (25, 125) in der Achsenrichtung eingestellt wird;
der Einsteller (25, 125) durch eine Schraubeneingriffsstruktur in der Achsenrichtung der Nadelbasis (21) bewegbar ist;
**dadurch gekennzeichnet, dass** der Bewegungsbetrag des Einstellers (25, 125) in Richtung der Oberseite durch einen Anschlagmechanismus (51, 53) auf einen vorbestimmten Betrag begrenzt ist;
der Anschlagmechanismus (51, 53) aus einem Kragenabschnitt (51) auf der Seite des Einstellers (25, 125), der mit einem Ende eines männlichen Schraubenabschnitts (33) auf der Seite der Nadelbasis (21) in Kontakt kommt, gebildet ist.

2. Injektionsnadel nach Anspruch 1, wobei das obere Ende der Nadel (23, 123) in den Einsteller (25, 125) zurückziehbar ist.

3. Injektionsnadel nach Anspruch 1, wobei eine Nadelbasis (21) mit einer Referenzpositionsmarkierung (40a) auf der Nadelbasisseite versehen ist und der Einsteller (25, 125) mit einer Referenzpositionsmarkierung (40b) auf der Einstellerseite versehen ist.

4. Injektionsnadel nach Anspruch 1, wobei
der Einsteller (25, 125) mit einer in Längsrichtung geteilten Nut (55) versehen ist, um eine Erweiterung des Durchmessers beim Anbringen an der Nadelbasis (21) zu ermöglichen.

5. Injektionsnadel nach Anspruch 1, wobei die Querschnittsform des männlichen Schraubenabschnitts (33) der Nadelbasis (21) eine nicht kreisförmige Form aufweist und die Querschnittsform einer Öffnung des Kragenabschnitts (51) des Einstellers entsprechend ebenfalls eine nicht kreisförmige Form aufweist, und durch Übereinstimmen jeder Position in Drehrichtung kann der männliche Schraubenteil (33) durch das Innere des Einstellers (25, 125) hindurchdringen.

6. Injektionsnadel nach einem der Ansprüche 1 bis 5, wobei die Nadel (23, 123) in einer Mehrzahl vorgesehen ist.

7. Injektionsnadel nach Anspruch 6, wobei ein Medikamentenauslass (23, 123a) der Nadel (23, 123) in Radialrichtung der Nadelbasis (21) nach innen ausgerichtet ist.

8. Injektionsnadel nach einem der Ansprüche 1 bis 7, wobei die Nadelbasis (21) mit einem Haltevorsprung versehen ist.

9. Injektionsnadel nach einem der Ansprüche 1 bis 8, wobei der Außenumfang des Einstellers (25, 125) mit einer Kappe (41, 201) gekrönt ist, um die Nadel abzudecken.

10. Injektionsnadel nach Anspruch 9, wobei
die Kappe (41) in den Einsteller eingesetzt ist, so dass der Einsteller (25, 125) mit der Drehung der Kappe (41) gedreht werden kann.

11. Injektionsnadel nach Anspruch 10, wobei
der Einsteller (25, 125) mit einem Eingriffsvorsprung (45) versehen ist und die Kappe (41) mit einer Eingriffsöffnung versehen ist, die mit dem Eingriffsvorsprung (45) in Eingriff bringbar ist.

12. Die Injektionsnadel nach Anspruch 9, wobei die Kappe den Einsteller umschließt und in der Nadelbasis in einem Zustand eingesetzt ist, in dem eine Drehung verhindert ist.

## Revendications

1. Aiguille d'injection munie de :
une aiguille (23, 123) ;
une base d'aiguille (21) maintenant l'aiguille (23, 123) dans un état de saillie vers le côté extrémité supérieure ; et un ajusteur (25, 125) disposé pour être mobile le long de la direction d'axe de la base d'aiguille (21) sur la périphérie extérieure de la base d'aiguille (21), dans laquelle une longueur de saillie de l'aiguille (23, 123) est ajustée en ajustant une position de l'ajusteur (25, 125) dans la direction d'axe ;
l'ajusteur (25, 125) est mobile dans la direction d'axe de la base d'aiguille (21) par une structure de mise en prise à vis ;
**caractérisée en ce que**
la quantité de déplacement de l'ajusteur (25, 125) vers le côté extrémité supérieure est limitée à une quantité prédéterminée par un mécanisme de butée (51, 53) ;
le mécanisme de butée (51, 53) est composé d'une partie de collier (51) sur le côté de l'ajusteur (25, 125) qui entre en contact avec une extrémité d'une partie de vis mâle (33) sur le côté de la base d'aiguille (21).

2. Aiguille d'injection selon la revendication 1, dans laquelle l'extrémité supérieure de l'aiguille (23, 123) est capable d'être rétractée dans l'ajusteur (25, 125).

3. Aiguille d'injection selon la revendication 1, dans laquelle une base d'aiguille (21) est munie d'un marqueur de position de référence côté base d'aiguille (40A), et l'ajusteur (25, 125) est muni d'un marqueur de position de référence côté ajusteur (40B).

4. Aiguille d'injection selon la revendication 1, dans laquelle,
l'ajusteur (25, 125) est muni d'une rainure fendue longitudinalement (55), de manière à permettre l'expansion du diamètre lors de la fixation à la base d'aiguille (21).

5. Aiguille d'injection selon la revendication 1, dans laquelle la forme en coupe transversale de la partie de vis mâle (33) de la base d'aiguille (21) est d'une forme non circulaire, et la forme en coupe transversale d'une ouverture de la partie de collier (51) de l'ajusteur est également par conséquent d'une forme non circulaire, et en faisant correspondre chaque position dans la direction de rotation, la partie de vis mâle (33) devient capable de pénétrer à travers l'intérieur de l'ajusteur (25, 125).

6. Aiguille d'injection selon l'une quelconque des revendications parmi les revendications 1 à 5, dans laquelle l'aiguille (23, 123) est fournie en nombre pluriel.

7. Aiguille d'injection selon la revendication 6, dans laquelle une sortie de solution médicinale (23, 123a) de l'aiguille (23, 123) est orientée vers l'intérieur dans la direction radiale de la base d'aiguille (21).

8. Aiguille d'injection l'une quelconque des revendications parmi les revendications 1 à 7, dans laquelle la base d'aiguille (21) est munie d'une saillie de maintien.

9. Aiguille d'injection selon l'une quelconque des revendications parmi les revendications 1 à 8, dans laquelle la périphérie extérieure de l'ajusteur (25, 125) est coiffée d'un capuchon (41, 201) de manière à recouvrir l'aiguille.

10. Aiguille d'injection selon la revendication 9, dans laquelle,
le capuchon (41) est monté dans l'ajusteur, de sorte que l'ajusteur (25, 125) peut être tourné avec la rotation du capuchon (41).

11. Aiguille d'injection selon la revendication 10, dans laquelle,
l'ajusteur (25, 125) est muni d'une saillie de mise en prise (45), et le capuchon (41) est muni d'une ouverture de mise en prise à mettre en prise avec la saillie de mise en prise (45).

12. Aiguille d'injection selon la revendication 9, dans laquelle le capuchon entoure l'ajusteur et est monté dans la base d'aiguille dans un état tel que la rotation est interdite.
